# EUROPEAN PATENT APPLICATION

(11) **EP 1 130 092 A1**
(43) Date of publication of application: **05.09.2001**
(21) Application number: 00102973.5
(22) Date of filing: 14.02.2000
(51) Int. Cl.: C12N 15/10, C12Q 1/68

(54) **A method of preparing recombinant nucleic acids**

(71) Applicant: Hosch, Stefan B., c/o Universität Hamburg, Abt. für Allgemeinchirurgie, 20246 Hamburg (DE); Kalinin, V., c/o Universität Hamburg, Abt. für Allgemeinchirurgie, 20246 Hamburg (DE); Izbicki, Jakob R., c/o Universität Hamburg, Abt. für Allgemeinchirurgie, 20246 Hamburg (DE)
(72) Inventor: Hosch, Stefan B., c/o Universität Hamburg, Abt. für Allgemeinchirurgie, 20246 Hamburg (DE); Kalinin, V., c/o Universität Hamburg, Abt. für Allgemeinchirurgie, 20246 Hamburg (DE); Izbicki, Jakob R., c/o Universität Hamburg, Abt. für Allgemeinchirurgie, 20246 Hamburg (DE)
(74) Representative: Schüssler, Andrea, Dr.

(57) **Abstract**

Described is a method of preparing recombinant nucleic acids, which comprises the steps of:
(a) amplification of a 1st nucleic acid fragment (x) in the presence of a polymerization agent, the various deoxynucleoside triphosphates, and two primers (x1/x2) hybridizing to form opposite strands of the nucleic acid, wherein after being separated from its template strand the extension product synthesized by one primer (xl) serves as a template for the synthesis of an extension product of the other primer (x2), the primer (x2) determining the terminus, to be linked, of the 1st nucleic acid and the 3' end of primer (x2) being substantially complementary to a strand of the desired terminus of the 1st nucleic acid and the 5' end of primer (x1) being not complementary to this strand and containing a recognition sequence for a type IIS restriction endonuclease;
(b) amplification of a 2nd nucleic acid fragment (y) in the presence of a polymerization agent, the various deoxynucleoside triphosphates and two primers (y1/y2) hybridizing to form opposite strands of the nucleic acid, wherein after being separated from its template strand the extension product synthesized by one primer (y1) serves as a template for the synthesis of an extension product of the other primer (y2), the primer (y1) determining the terminus, to be linked, of the 2nd nucleic acid and the 3' end of primer (y1) being substantially complementary to a strand of the desired terminus of the 2nd nucleic acid and the 5' end of primer (y1) being not complementary to this strand and containing a recognition sequence for a type IIS restriction endonuclease; wherein
(c) a sequence identical in both primers is additionally present between this 3' end and the 5' end of primers (x2) and (y1), which corresponds to the sequence cleaved by the restriction enzyme;
(d) restriction of the amplified 1st and 2nd nucleic acids with (a) restriction endonuclease(s) recognizing the recognition sequences defined in (a) and (b); and
(e) ligation of the nucleic acid fragments obtained in (d).

## Description

The present invention relates to a method of preparing recombinant nucleic acids.

The preparation of recombinant genes is widely used both in research and for commercial purposes, e.g. for the preparation of expression vectors to produce recombinant proteins. For the preparation of recombinant genes, the 1^{st} parental gene and the 2^{nd} one what is just cloned into the vector are usually cleaved with suitable restriction endonucleases, and these fragments are joined by means of ligase. After propagation of the vector carrying the inserted gene in host bacteria, it can be sequenced so as to control, for example, whether the transitions between vector and insertion have the expected sequence. However, this procedure comprises a number of drawbacks, one of the greatest drawbacks being that suitable recognition sequences for restriction endonucleases on the gene to be inserted are not always present at the desired positions. Recognition sequences for the most frequently used restriction endonucleases having palindromic hexanucleotide recognition sites occur on the average only once in several thousand base pairs. In this connection, the cleavage sites are within the recognition sequence.

There are some methods which solve the problem as to the recognition sequence for restriction endonucleases, suitable for cloning, but only do this partially. They are based e.g. on the joining of fragments with synthetic oligonucleotides which have the desired recognition sequence and restriction site, respectively. For adapting the desired nucleotide sequence of the recombinant gene and retaining the correct reading frame, methods are also used where an unspecific cleavage of DNA fragments with exonucleases and/or endonucleases is applied. However, all of these methods are accompanied by considerable drawbacks. For example, they are rather complex and time-consuming and cannot be applied generally to the construction of any recombinant nucleic acid sequences.

Thus, the present invention is based on the technical problem of providing a method of preparing recombinant nucleic acid sequences, which does not comprise the drawbacks of the methods described in the prior art, i.e. enables above all accurate and simple joining of any nucleic acids and is generally applicable.

This technical problem is solved by the provision of the embodiments characterized in the claims.

The method according to the invention is based on some of the following properties of type IIS restriction endonucleases:
(a) They recognize non-palindromic DNA sequences and cleave at a certain distance outside the recognition site, wherein the cleavage sequence may comprise any sequence and is only determined by the distance from the recognition sequence;
(b) A number of different type IIS restriction endonucleases are known by now, which recognize different non-palindromic nucleic acid sequences, the length of the recognition sequence being 4 to 7 base pairs and blunt ends or overhanging single-stranded ends having a length of 1 to 6 nucleotides being produced. Since the overhanging ends produced by these restriction enzymes and localized in certain regions outside the recognition sequences are "unique", the desired nucleic acids can be joined in well-calculated fashion and accurately by means of the below described procedure.

Primer (x1)....ATGACT...1.nucleic acid..CGAATATGATCCAACAATAGAGtgaagagcgggttt
...............TACTGA...1.nucleic acid..GCTTATACTAGGTTGTTATCTCACTTCTCGCCCAAA-5'
Primer (x2)
Primer (y1)
5'-AAACCCGCTCTTCAGAGGATTCCTACAGGAAGCAAGT...2.nucleic acid.TTTGAA..........
tttgggcgagaagtctcCTAAGGATGTCCTTCGTTCT...2.nucleic acid.AAACTT...Primer (y2)

New hybrid nucleic acid after restriction of the two amplified fragments and subsequent ligation:

5'-...CGAATATGATCCAACAATAGAG|GATTCCTACAGGAAGCAAGT...-3'

Sequences emphasized by bold-face: primer sequences Arrows show the cleavage points for SapI Sequences written in italics: restriction site for SapI Underlined sequences: recognition sequence for SapI Small letters: sequences which were not originally present on the template and were generated by (non-complementary) primer sequences during the amplification
|: linking site between 1st and 2nd nucleic acids

Thus, the present invention relates to a method of preparing recombinant nucleic acids, which comprises the steps of:
(a) amplification of a 1st nucleic acid fragment (x) in the presence of a polymerization agent, the various deoxynucleoside triphosphates and two primers (x1/x2) hybridizing to form opposite strands of nucleic acid, wherein after being removed from its template strand, the extension product synthesized by a primer (x1) serves as a template for the synthesis of an extension product of the other primer (x2), primer (x2) determining the terminus, to be linked, of the 1st nucleic acid and the 3' end of primer (x2) being substantially complementary to a strand of the desired terminus of the 1st nucleic acid and the 5' end of primer (x1) being not complementary to this strand and containing a recognition sequence for a type IIS restriction endonuclease;
(b) amplification of a 2nd nucleic acid fragment (y) in the presence of a polymerization agent, the various deoxynucleoside triphosphates and two primers (y1/y2) hybridizing to form opposite strands of the nucleic acid, wherein after being removed from its template strand, the extension product synthesized by a primer (y1) serves as a template for the synthesis of an extension product of the other primer (y2), the primer (y1) determining the terminus, to be linked, of the 2nd nucleic acid and the 3' end of primer (y1) being substantially complementary to a strand of the desired terminus of the 2nd nucleic acid and the 5' end of primer (y1) being not complementary to this strand and containing a recognition sequence for a type IIS restriction endonuclease; wherein
(c) a sequence identical in both primers is additionally present between this 3' end and the 5' end of primers (x2) and (y1), which corresponds to the sequence cleaved by the restriction enzyme;
(d) restriction of the amplified 1st and 2nd nucleic acids with (a) restriction endonuclease(s) recognizing the recognition sequences defined in (a) and (b); and
(e) ligation of the nucleic acid fragments obtained in (d).

Steps (a) to (e) can be carried out by means of methods known in this field, as described e.g. in Ausubel and Frederick (1991), Current Protocols in Molecular Biology (J. Wiley & Sons, New York). For example, polymerase chain reaction (PCR) can be carried out for the amplification, as described e.g. in EP-B1 0 201 184. Suitable reaction conditions, e.g. as regards the temperatures and duration of the individual PCR cycles, primer concentrations, polymerization agents (e.g. Taq-DNA polymerase), etc., are known to a person skilled in the art and also described in EP-B1 0 201 184. It is preferred to use as polymerization agents DNA polymerases which have proofreading activity so as to ensure the accuracy of the amplification (e.g. Lundberg et al., Gene 108, p. 1-6 (1991)).

In the amplification of the individual DNA fragments to be ligated, the primers having the above described properties must be used in the termini of the nucleic acids which shall accurately be ligated with one another. As to the termini which shall not be ligated with one another (left-hand terminus of the 1st nucleic acid and right-hand terminus of the 2nd nucleic acid in the above scheme), it is possible to use common primers, e.g. primers which are fully complementary to the desired strand of the template. These primers can also comprise e.g. a 5' end which is not complementary to the template and contains a recognition sequence for a restriction endonuclease thus enabling ligation in a vector cleaved by the appropriate restriction enzyme, e.g. an expression vector, after amplification.

The expression "substantially complementary" used herein must be comprehended such that in a ligation of the desired DNA fragments preferably primers are used which are fully complementary to the template; in a ligation accompanied by the simultaneous insertion of mutations in the neighborhood of the ligation site, it is possible to use primers which are not fully complementary to the template - corresponding to the desired mutation.

The sequence localized between the above described 3' end and 5' end of the primers and corresponding to one strand of the restriction sequence for the type IIS restriction endonuclease, is preferably complementary to the template sequence in the case of one primer, e.g. primer (x2) and not complementary to the template sequence in the case of the other primer, e.g. primer (y1). In the latter case, a cleavage sequence is introduced by this primer at the desired position within the nucleic acid fragment to be ligated. As shown in the above scheme, this procedure serves for ensuring that following amplification and restriction the nucleic acids are ligated accurately with one another, i.e. no substitution, deletion or addition of one or more base pairs forms at the ligation site.

In a preferred embodiment, the method according to the invention relates to the preparation of recombinant nucleic acids, where at least two nucleic acids are ligated with each other. For example, if three or more nucleic acids shall be joined, the procedure given in the above scheme can be varied correspondingly, e.g. as indicated in the below example.

In a particularly preferred embodiment of the method according to the invention, the amplification takes place via PCR, as described e.g. in EP-B1 0 201 184.

In principle, the method according to the invention is not restricted to the ligation of any DNA fragments but also enables the ligation of single-stranded DNAs or RNAs. In this connection, the single-stranded nucleic acids are converted into double-stranded DNA by means of generally known methods prior to steps (a) and (b), respectively, e.g. in the case of mRNAs the 1st cDNA strand is synthesized by means of an oligo(dT) or random primers and reverse transcriptase and the second DNA strand is synthesized e.g. by RNase digestion followed by PCR. Here, RNA artifacts are used as starting points, which were produced by RNaseH. Thus, another preferred embodiment of the method according to the invention is characterized in that the nucleic acids to be ligated are single-stranded DNAs or RNAs which are converted into a double-stranded DNA prior art step (a) and (b), respectively.

In another preferred embodiment of the method according to the invention, the recognition sequences for the restriction endonuclease(s) have a length of 4 to 7 base pairs, recognition sequences having a length of 7 base pairs being preferred, since this minimizes the probability that the DNA fragments to be ligated accidentally have, at undesired sites, a recognition sequence, identical with the recognition sequence comprised by the primers.

All of the type IIS restriction endonucleases can be used for the method according to the invention, irrespective of whether they produce blunt ends or overhanging ends. However, restriction endonucleases producing overhanging ends having a length of 1 to 6 bases are preferred. Especially preferred type IIS restriction endonucleases are SapI, BsrDI or BseRI wherein BsrDI and BseRI do not only produce overhanging 5' ends but also overhanging 3' ends.

The length of the 3' end of the primers of the method according to the invention is not critical so long as they provide a specificity for amplification. The preferred length is 14 to 20 bases. As a rule, a person skilled in the art will chose a length ensuring that the primers can attach stably and specifically to the template. However, the method according to the invention preferably uses primers where the 3' ends which are substantially complementary to the template contain at least 15 to 25 bases.

The length of the 5' ends of the primers used in the method according to the invention is not critical either and depends inter alia on the length of the recognition sequence of the restriction endonuclease.

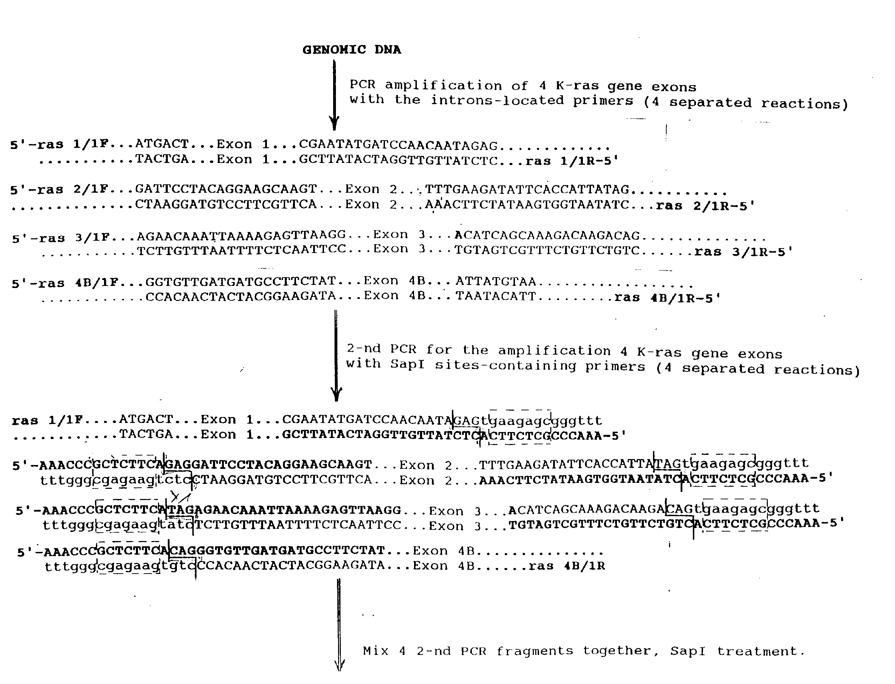

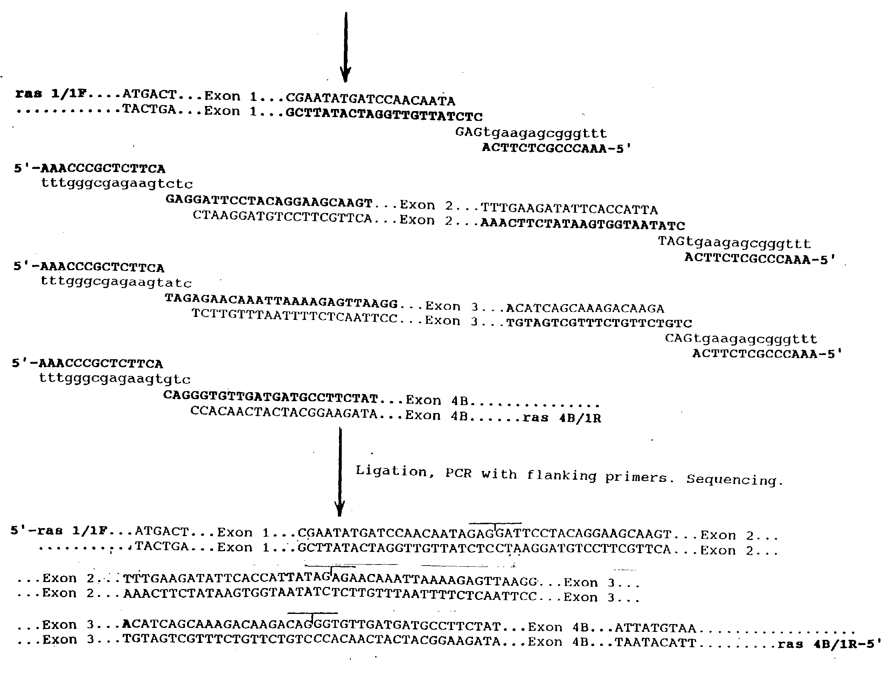

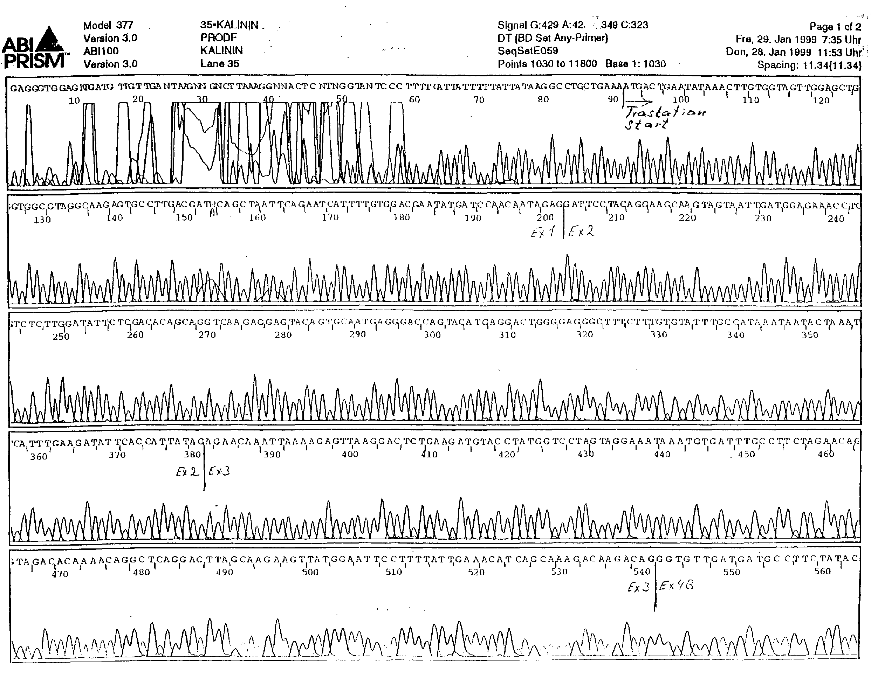

The method according to the invention can be used for the most differing purposes in which accurate ligation of nucleic acids at certain defined positions is desired, e.g. for joining DNA fragments coding for differing domains of a protein by retaining the reading frame to produce chimeric or humanized antibodies or to attach an extra N-end amino acid sequence for secretion ot to attach regulatory elements (e.g. promoter, polyA-signal, terminator of translation etc.) to genes. A particular cause and an illustration for the application of the method according to the invention consists in the production of intron-free genes, it being possible to take the steps shown in the scheme of figure 1.

This procedure, which is of interest e.g. in the recombinant expression of human genes in prokaryotes, costs much less and is considerably less time-consuming than the current methods of producing intron-free genes which comprise e.g. the isolation of cDNA and the conversion into double-stranded DNA by means of reverse transcriptase.

Finally, the present invention relates to a kit for carrying out the above described method, which contains the above described primers. The kit may contain further components which are required for carrying out the entire method, e.g. a suitable DNA polymerase, ligase, restriction enzyme(s) of type IIS and dNTPs and optionally also the enzymes and primers required for converting single-stranded DNAs or RNAs into double-stranded DNAs.

The present invention has the following important advantages:
- It provides a possibility to make recombinant genes starting from a unique gene without prior cloning
- All manipulations including the analysis of a recombinant product of interest by sequencing can be carried out in vitro without cloning in living organisms (e.g. bacteria). This makes the method fast and without a risk for environment.
- In view of a unique structure of "sticky ends" produced by type IIS restriction enzyme cleavage it is possible to get a correct joining of all fragments in a single ligase reaction. This is the reason why "naturally" occurring type IIS restriction sites in the fragments do not destroy the reaction. It is not necessary to protect "naturally" occurring sites by methylation, e.g. via introduction of the highly mutagenic 5-methyl-desoxycytosine into the PCR as described by Padgett et al., Gene 168, pp. 31-35 (1996).
- With the present method only very few PCR mistakes are observed. However, if such mistakes occur, it is easily possible to find a clone without mistakes or to correct one or more ones by a procedure similar to the present invention.

The invention is described in more detail with reference to the figures.

Figure 1: Diagram of the method according to the invention
1. Amplification of the 4 exons of the human K-ras gene by means of the following primers: 
Exon 1: 5'-GTACTGGTGGAGTATTTGATAG (ras 1/1F) and
5'-ACTCATGAAAATGGTCAGAGAAAC (ras 1/1R)
Exon 2: 5'-GTTGTTGACTTGTATATAACACCT (ras 2/1F) and
5'-CCAAGAACTTCATTTATAAAACAG (ras 2/1R)

Exon 3: 5'-ACTAGTAGAAGAAGGAAGGAAA (ras 3/1F) and
5'-TATTAAATGACATAACAGTTATGA (ras 3/1R)
Exon 4B: 5'-GTTACCTGTACACATGAAGC (ras 4B/1F) and
5'-GGAATACTGGCACTTCGAGG (ras 4B/1R).
2. Amplification by SapI primers:

Exon 1: 5'-GTACTGGTGGAGTATTTGATAG (ras 1/1F) and
5'-AAACCCGCTCTTCACTCTATTGTTGGATCATATTCG (SP1-R)
Exon 2: 5'-AAACCCGCTCTTCAGAGGATTCCTACAGGAAGCAAGT (SP2-F) and
5'-AAACCCGCTCTTCACTATAATGGTGAATATCTTCAAA (SP2-R)
Exon 3: AAACCCGCTCTTCATAGAGAACAAATTAAAAGAGTTAAGG (SP3-F) and
5'-AAACCCGCTCTTCACTGTCTTGTCTTTGCTGATGT (SP3-R)
Exon 4B: 5'-AAACCCGCTCTTCACAGGGTGTTGATGATGCCTTCTAT (SP4-F) and
5'-GGAATACTGGCACTTCGAGG (ras 4B/1R).

### Figure 2: Checking the accuracy of the removal, described in the example, of the introns from the K-ras gene

The nucleic acid obtained according to the example was sequenced by means of BigDye dideoxy-termination nucleotides and an "ABI 377 automatic sequencer" (Perkin Elmer Applied Biosystems (Weiterstadt, Germany). The figure shows that the exons were ligated by accurately retaining the correct reading frame. The exon/exon transitions are marked.

The below example explains the invention.

### Example: "Artificial" splicing of the human K-ras gene

The steps, carried out in this example, of the first amplification and the second amplification by SapI primers are shown in figure 1.

### (A) 1st PCR amplification of the 4 exons of the K-ras gene by primers localized in intron regions

Human genomic DNA obtained by standard methods from cell line K562 (Gibco-BRL Life Technologies, Eggenstein, Germany) served as a template for the 1st amplification reaction. The PCR amplifications were carried out in 4 separate batches in the following reaction mixture each: 100 *µ*l mM Tris-HCl (pH value 8.8), 1.5 mM MgCl2, 25 mM KCl, 100 nM dNTPs, 0.5 *µ*g sample-DNA, 2 *µ*l primer (25 pmol/µl), 0.5 µl (2.5 U) Taq DNA polymerase. The reaction conditions were as follows: 2 min. denaturation at 95°C, thereafter 30 PCR cycles (30 sec. at 95°C; 30 sec. at 55°C, 1 min. at 72°C). Extension at 72°C for 2 min. For reducing the possibility of forming PCR artifacts, a DNA polymerase without proofreading activity was used alternatively (Pfu DNA polymerase).

### (B) 2nd PCR amplification by primers containing a recognition sequence for SapI

2 µl of the particular reaction mixture with amplified nucleic acids from (A) were added in each case to a reaction mixture which corresponded to that of (A), with the exception that in place of the primers indicated for (A) in the legend (top) of figure 1 the SapI primers pairs indicated in the legend (bottom) of figure 1 were contained in the mixture per reaction mixture. The reaction conditions also corresponded to those conditions indicated in (A), but only 15 PCR cycles were performed.

### (C) SapI restriction and ligation

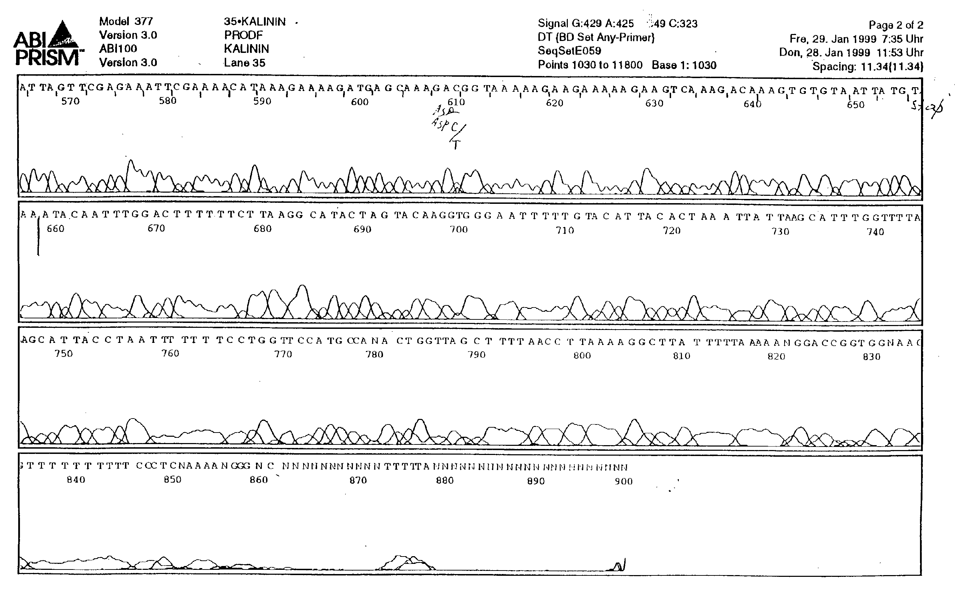

25 *µ*l of each of the four reaction mixtures with the amplified nucleic acids from (B) were mixed and purified by means of "QIAquick PCR Purification Kit" (Qiagen, Hilden, Germany) in accordance with the manufacturer's instructions. 25 *µ*l of the mixture with the purified fragments were then cleaved by 5 units of SapI restriction endonuclease (New England Biolabs, Schwalbach, Germany] at 37°C for 3 hours, and then the enzyme was inactivated at 70°C for 15 minutes. Thereafter, 20 *µ*l of the mixture with the SapI fragments were treated with 100 units T4 ligate (New England Biolabs) at 14°C for 16 hours. 1 *µ*l of the mixture with the ligated fragments was amplified again (100 *µ*l reaction volume, composition of the mixture as under (A) with the exception that the primer pair "ras 1/1F" and "ras 4B/1R" was used). The reaction conditions were as follows: 2 minutes of denaturation at 95°C, thereafter 15 PCR cycles (30 sec. at 95°C; 30 sec. at 55°C; 2 min. at 72°C). Extension 2 min. at 72°C. The PCR product was purified again with the "QIAquick PCR Purification Kit" (Qiagen) and sequenced with "BigDye" dideoxy termination nucleotides and an "ABI 377 automatic sequencer" (Perkin Elmer Applied Biosystems, Weiterstadt, Germany). The sequence illustrated in figure 2 shows clearly that the ligation of the individual fragments took place as desired, i.e. the exon-exon transitions were correct.

## Claims

1. A method of preparing recombinant nucleic acids, which comprises the steps of:
(a) amplification of a 1st nucleic acid fragment (x) in the presence of a polymerization agent, the various deoxynucleoside triphosphates, and two primers (x1/x2) hybridizing to form opposite strands of the nucleic acid, wherein after being separated from its template strand the extension product synthesized by one primer (x1) serves as a template for the synthesis of an extension product of the other primer (x2), the primer (x2) determining the terminus, to be linked, of the 1st nucleic acid and the 3' end of primer (x2) being substantially complementary to a strand of the desired terminus of the 1st nucleic acid and the 5' end of primer (x1) being not complementary to this strand and containing a recognition sequence for a type IIS restriction endonuclease;
(b) amplification of a 2nd nucleic acid fragment (y) in the presence of a polymerization agent, the various deoxynucleoside triphosphates and two primers (y1/y2) hybridizing to form opposite strands of the nucleic acid, wherein after being separated from its template strand the extension product synthesized by one primer (y1) serves as a template for the synthesis of an extension product of the other primer (y2), the primer (y1) determining the terminus, to be linked, of the 2nd nucleic acid and the 3' end of primer (y1) being substantially complementary to a strand of the desired terminus of the 2nd nucleic acid and the 5' end of primer (y1) being not complementary to this strand and containing a recognition sequence for a type IIS restriction endonuclease; wherein
(c) a sequence identical in both primers is additionally present between this 3' end and the 5' end of primers (x2) and (y1), which corresponds to the sequence cleaved by the restriction enzyme;
(d) restriction of the amplified 1st and 2nd nucleic acids with (a) restriction endonuclease(s) recognizing the recognition sequences defined in (a) and (b); and
(e) ligation of the nucleic acid fragments obtained in (d) .

2. The method according to claim 1, wherein at least two nucleic acids are ligated with one another.

3. The method according to claim 1 or 2, wherein the amplification is a polymerase chain reaction (PCR).

4. The method according to any one of claims 1 to 3, further **characterized in that** the nucleic acids to be ligated are single-stranded DNAs or RNAs which are converted into a double-stranded DNA prior to step (a) and (b), respectively.

5. The method according to any one of claims 1 to 4, wherein the recognition sequences defined in (a) and (b) are identical for the restriction endonuclease.

6. The method according to any one of claims 1 to 5, wherein the recognition sequences for the restriction endonuclease have a length of 4 to 7 base pairs.

7. The method according to claim 6, wherein the recognition sequences for the restriction endonucleases have a length of 7 base pairs.

8. The method according to any one of claims 1 to 7, wherein the restriction endonuclease produces overhanging ends having a length of 1 to 6 bases.

9. The method according to claim 8, wherein the 3' ends of the primers which are substantially complementary to the template, contain at least 15 to 25 bases.

10. The method according to any one of claims 1 to 9, wherein the restriction endonculease is SapI.

11. Use of the method according to any one of claims 1 to 10 for producing intron-free nucleic acids.

12. Kit for carrying out the method according to any one of claims 1 to 11, containing the primers defined in claims 1 to 9.


### Annex to the application documents - subsequently filed sequences listing

SEQUENCE LISTING

<110> Hosch, Stefan et al.

<120> A Method of Preparing Recombinant Nucleic Acids

<130> H 1010EU

<140> EP 00 102 973.5
<141> 2000-02-15

<160> 15

<170> PatentIn Ver. 2.1

<210> 1
<211> 640
<212> DNA
<213> Artificial Sequence

<220>
<223> Description of Artificial Sequence: Artifically
      sliced human K-ras gene

<400> 1

ttttcattat ttttattata aggcctgctg aaaatgactg aatataaact tgtggtagtt 60
ggagctggtg gcgtaggcaa gagtgccttg acgatacagc taattcagaa tcattttgtg 120
gacgaatatg atccaacaat agaggattcc tacaggaagc aagtagtaat tgatggagaa 180
acctgtctct tggatattct cgacacagca ggtcaagagg agtacagtgc aatgagggac 240
cagtacatga ggactgggga gggctttctt tgtgtatttg ccataaataa tactaaatca 300
tttgaagata ttcaccatta tagagaacaa attaaaagag ttaaggactc tgaagatgta 360
cctatggtcc tagtaggaaa taaatgtgat ttgccttcta gaacagtaga cacaaaacag 420
gctcaggact tagcaagaag ttatggaatt ccttttattg aaacatcagc aaagacaaga 480
cagggtgttg atgatgcctt ctatacatta gttcgagaaa ttcgaaaaca taaagaaaag 540
atgagcaaag acggtaaaaa gaagaaaaag aagtcaaaga caaagtgtgt aattatgtaa 600
atacaatttg actttttctt taaggcatac tagtacaagt 640

<210> 2
<211> 22
<212> DNA
<213> Artificial Sequence

<220>
<223> Description of Artificial Sequence: Primer

<400> 2

gtactggtgg agtatttgat ag 22

<210> 3
<211> 24
<212> DNA
<213> Artificial Sequence

<220>
<223> Description of Artificial Sequence: Primer

<400> 3

actcatgaaa atggtcagag aaac 24

<210> 4
<211> 24

<212> DNA
<213> Artificial Sequence

<220>
<223> Description of Artificial Sequence: Primer

<400> 4

gttgttgact tgtatataac acct 24

<210> 5
<211> 24
<212> DNA
<213> Artificial Sequence

<220>
<223> Description of Artificial Sequence: Primer

<400> 5

ccaagaactt catttataaa acag 24

<210> 6
<211> 22
<212> DNA
<213> Artificial Sequence

<220>
<223> Description of Artificial Sequence: Primer

<400> 6

actagtagaa gaaggaagga aa 22

<210> 7
<211> 24
<212> DNA
<213> Artificial Sequence

<220>
<223> Description of Artificial Sequence: Primer

<400> 7

tattaaatga cataacagtt atga 24

<210> 8
<211> 20
<212> DNA
<213> Artificial Sequence

<220>
<223> Description of Artificial Sequence: Primer

<400> 8

gttacctgta cacatgaagc 20

<210> 9
<211> 20
<212> DNA

<213> Artificial Sequence

<220>
<223> Description of Artificial Sequence: Primer

<400> 9

ggaatactgg cacttcgagg                                                    20


<210> 10
<211> 36
<212> DNA
<213> Artificial Sequence

<220>
<223> Description of Artificial Sequence: Primer

<400> 10

aaacccgctc ttcactctat tgttggatca tattcg                                  36


<210> 11
<211> 37
<212> DNA
<213> Artificial Sequence

<220>
<223> Description of Artificial Sequence: Primer

<400> 11

aaacccgctc ttcagaggat tcctacagga agcaagt                                 37


<210> 12
<211> 37
<212> DNA
<213> Artificial Sequence

<220>
<223> Description of Artificial Sequence: Primer

<400> 12

aaacccgctc ttcactataa tggtgaatat cttcaaa                                 37


<210> 13
<211> 40
<212> DNA
<213> Artificial Sequence

<220>
<223> Description of Artificial Sequence: Primer

<400> 13

aaacccgctc ttcatagaga acaaattaaa agagttaagg                              40


<210> 14
<211> 35
<212> DNA
<213> Artificial Sequence

<220>

<223> Description of Artificial Sequence: Primer

<400> 14

aaacccgctc ttcactgtct tgtctttgct gatgt                                35

<210> 15
<211> 38
<212> DNA
<213> Artificial Sequence

<220>
<223> Description of Artificial Sequence: Primer

<400> 15

aaacccgctc ttcacagggt gttgatgatg ccttctat                             38